# EUROPÄISCHE PATENTSCHRIFT

(11) **EP 2 790 596 B1**
(45) Veröffentlichungstag und Bekanntmachung des Hinweises auf die Patenterteilung: **03.06.2015**
(21) Anmeldenummer: 13718124.4
(22) Anmeldetag: 18.04.2013
(51) Int. Cl.: A61B 17/32, A61B 17/16, A61B 17/00, A61B 1/313, A61B 1/012, A61B 1/00

(54) **CHIRURGISCHE WERKZEUGEINRICHTUNG**
SURGICAL TOOL DEVICE
DISPOSITIF D'OUTIL CHIRURGICAL

(30) Priorität: 03.05.2012 DE 102012008970
(43) Veröffentlichungstag der Anmeldung: 22.10.2014
(73) Patentinhaber: Joimax GmbH, 76227 Karlsruhe (DE)
(72) Erfinder: RIES, Wolfgang, 76351 Linkenheim (DE); KRAUSEMANN, Achim, 76185 Karlsruhe (DE)
(74) Vertreter: Lempert, Jost
(86) Internationale Anmeldenummer: PCT/EP2013/001151
(87) Internationale Veröffentlichungsnummer: WO 2013/164067

(56) Entgegenhaltungen:
- DE-A1- 19 533 856
- US-A1- 2004 244 806
- US-A1- 2009 221 873
- US-A1- 2011 112 359
- US-B1- 6 196 967

## Beschreibung

Die Erfindung betrifft eine chirurgische Werkzeugeinrichtung nach dem Oberbegriff des Anspruchs 1.

Derartige chirurgische Werkzeugeinrichtungen dienen zum Arbeiten in Gewebe, insbesondere Knochengewebe, wie im Wirbelbereich, beispielsweise um dort auf Nerven treffendes Gewebe, auch Bandscheibengewebe, zu entfernen oder aber Voraussetzungen zur Stabilisierung, wie zur Fixierung zweier Wirbel, zu schaffen. Einzusetzende Werkzeuge sind rotatorisch betriebene Werkzeuge, wie Bohrer oder Fräser. Die Einbringung geschieht mikroinvasiv endoskopisch. Oft ist es nur sehr schwer oder nicht möglich, eine Werkzeugeinrichtung mikroinvasiv endoskopisch derart zum Arbeitsort einzuführen, dass dieser in der Flucht des Einführkanals und auch der Achse der Werkzeugeinrichtung liegt, sei es, dass auf diesem Weg empfindliche Organe, Blutgefäße oder Nerven gefährdet sind. Es ist daher gewünscht oder auch notwendig, einen Arbeitskanal zu wählen, an dessen Ende der Arbeitsort dann außer der Flucht des Arbeitskanals und damit auch der Werkzeugachse bzw. versetzt hierzu liegt.

Die US 2011/112359 A1 zeigt eine chirurgische Adapteranordnung zur Verwendung mit einem Endoskop. Ein Schaft des Endoskops ist durch einen Endoskopkanal proximal mit einem Griffteil versehenem Trokar mit mehreren Kanälen hindurchschiebbar. Weiterhin sind durch das Endoskop Kanülen einer chirurgischen Schneideinrichtung hindurchschiebbar, wobei vom Chirurgen mittels eines Drehrads die äußere Kanüle des Schneidgeräts gedreht werden kann und vorzugsweise dann, wenn die äußere Kanüle sich dreht, eine innere Kanüle sich ebenso dreht, um eine feste angulare Orientierung zwischen äußerer und innerer Kanüle aufrechtzuerhalten. Dabei sind die Kanülen des chirurgischen Geräts zwar durch das Griffteil des Endoskops, nicht aber das Rohr (Schaft) desselben, sondern parallel zu letzterem durch einen separaten Arbeitskanal des Trokars einschiebbar.

Die US 6,196,967 B1 zeigt ein arthroskopisches System mit verbindbaren Komponenten, nämlich einem (Endo-)Skopkörper, einem mit diesem verbindbaren Hauptkörper mit einem Durchlass sowie alternativ einem mit diesem verbindbaren Brückenadapter oder rohrförmigen Skopmantel, die beide den vorgenannten Durchlass fortsetzen.

Ein Werkzeug selbst und die Verbindung mit einem solchen ist nicht angesprochen.

Die US 2009/0221873 A1 zeigt bei einem Adapter zur endoskopischen Orientierung eines länglichen medizinischen Geräts eine Nut-Federverbindung zweier Teile an sich.

Der Erfindung liegt daher die Aufgabe zugrunde, eine chirurgische Werkzeugeinheit mit einem rotatorisch betriebenen Werkzeug zu schaffen, die die Bearbeitung von Gewebe, insbesondere auch Knochengewebe, außerhalb der Flucht der Achse des Werkzeugs bzw. des Einführkanals ermöglicht. Dabei soll eine Flexibilität dahingehend gegeben sein, dass der Versatz nicht nur in einer Richtung möglich ist, sondern mit Orientierungen über den gesamten Umfang der Achse der Werkzeugeinrichtung.

Erfindungsgemäß wird die genannte Aufgabe mit einer gattungsgemäßen chirurgischen Werkzeugeinrichtung gelöst, die die kennzeichnenden Merkmale des Anspruch 1 aufweist. Das Führungsteil ist daher in einer Arbeitskonfiguration mit dem Endoskop fest verbindbar, zum Einführen und Herausnehmen aber auch wieder lösbar. Es sind hierzu eine Umfangsnut am proximalen Ende des Endoskops und eine am Führungsteil zum Eingriff in der Umfangsnut ausgebildete beweglich gelagerte Nase vorgesehen. Die Nase ist insbesondere an dem distalen Ende einer wippenartigen Klinke ausgebildet. Bei axial übereinstimmender Anordnung von Umfangsnut des Endoskops und der Nase des Führungsteils ist die Nase unter Einwirkung eines Übergangsteils der Führungskanüle in die Umfangsnut zur axialen Festlegung von Endoskop und Führungsteil eindrückbar.

Durch die Erfindung wird einerseits eine sichere und andererseits eine sowohl leicht herstellbare als auch leicht lösbare - oxide - Verbindung zwischen dem Endoskop und der Arbeitsgerätschaft aus zumindest Führungsteil und Werkzeugteil geschaffen. Es wird so insbesondere sichergestellt, dass dann, wenn der Werkzeugkopf des Werkzeugteils distal aus dem ihn zunächst voll umfassenden Mantelbereich des Führungsteils herausgeführt ist, eine sichere Verbindung von Endoskop und Führungsteil gegeben ist, die nur - einfach - lösbar ist, wenn der Werkzeugkopf in den umgebenden Mantel des Führungsteils zurückgezogen ist.

Das Führungsteil kann als Deflektor ausgebildet sein.

Durch einen Deflektor, der in seinem distalen Endbereich eine seitlich abgebogene Leitfläche aufweist und mit einem rotatorisch angetriebenen Werkzeugteil, das ebenfalls in seinem distalen Endbereich, unmittelbar proximal vor dem eigentlichen Werkzeug oder Werkzeugkopf flexibel ausgebildet ist, wird das Werkzeugteil beim Einschieben durch die Leitfläche des Deflektors an seinem flexiblen Endbereich zur Seite abgebogen und damit der Werkzeugkopf außerhalb der Flucht der Achse von Deflektor und Werkzeugteil gebracht, so dass hierdurch das gewünschte und angestrebte zur Achse versetzte Arbeiten bzw. zum Arbeiten außer der Flucht der Achse möglich ist.

In Weiterbildung ist dabei vorgesehen, dass das Werkzeugteil in seinem distalen Endbereich unmittelbar proximal des Werkzeugs eine Wendel aufweist.

In bevorzugter Ausgestaltung kann eine das Werkzeugteil aufnehmende Führungskanüle, welche mit dem Werkzeugteil innerhalb des Führungsteils aufgenommen ist, vorgesehen sein.

Wenn es gewünscht oder notwendig ist, wie bei der Ausgestaltung des Führungsteils als - abgebogener - Deflektor, Führungsteil und Führungskanüle des Werkzeugteils in eine geeignete feste umfangsseitige Ausrichtung zueinander zu bringen, ist in bevorzugter Ausgestaltung vorgesehen, dass die Führungskanüle proximal eine achsparallele Außennut und dass das Führungsteil innerhalb eines proximalen Verbindungsteils einen achsparallel gerichteten Stift zum Zusammenwirken mit der Nut aufweist, wobei insbesondere ein Rohr des Führungsteils relativ zu seinem Kupplungsteil entgegen der Wirkung einer Feder axial beweglich ist.

Weitere bevorzugte Ausgestaltungen der Erfindung sehen vor, dass Führungskanüle und Werkzeugteil axial fest, aber relativ zueinander drehbar miteinander verbunden sind und/oder dass das Werkzeugteil mit der Abtriebswelle eines Drehantriebs kuppelbar ist.

Wie schon angedeutet, kann insbesondere vorgesehen sein, dass das Führungsteil als Deflektor mit einem an seinem distalen Ende gegenüber seiner Achse seitlich abgebogenen Führungsabschnitt ausgebildet ist. Eine Weiterbildung sieht dann vor, dass sowohl Führungskanüle als auch Werkzeugteil in ihrem distalen Endbereich flexibel ausgebildet sind.

Die Führungskanüle ist bevorzugt in ihrem distalen Endbereich mit sich lediglich um einen Teil des Umfangs erstreckenden Schlitzen versehen, wobei insbesondere die Schlitze sich über 200° bis 300° erstrecken. Hierdurch wird in einfacher Weise ebenfalls die Flexibilität der Führungskanüle in ihrem Endbereich ermöglicht.

Da insbesondere bei der Ausgestaltung der vorstehenden konkreten Ausgestaltung der Führungskanüle deren Flexibilität nicht isotrop ist, sondern ihr Endbereich vorzugsweise zu der Richtung hin biegbar ist, auf der sich die Schlitze befinden (oder auch zur gegenüberliegenden Richtung hin) ist es notwendig Deflektor und Führungskanüle in der oben beschriebenen Weise eine feste umfangsmäßige Ausrichtung zueinander zu bringen, also insbesondere derart, dass die Schlitze auf der Seite der Kanüle angeordnet sind, die der Leitfläche des Deflektors gegenüber liegt.

Insgesamt wird durch die Erfindung eine chirurgische Werkzeugeinrichtung geschaffen, mit der der Operateur einfach und sicher notwendige Arbeiten, insbesondere spanabhebende Abtragungsarbeiten an Knochenmaterial, wie an Wirbeln, oder Gewebeabtragungen im Wirbelbereich vornehmen kann.

Weitere Vorteile und Merkmale der Erfindung ergeben sich aus den Ansprüchen und aus der nachfolgenden Beschreibung, in der ein Ausführungsbeispiel der Erfindung unter Bezugnahme auf die Zeichnung im Einzelnen erläutert ist. Dabei zeigt:
- Fig. 1: einen Längsschnitt durch eine bevorzugte Ausführungsform der erfindungsgemäßen Werkzeugeinheit gemäß A-A der Fig. 2;
- Fig. 2: eine Draufsicht auf die erfindungsgemäße Vorrichtung der Fig. 1 in Richtung des Pfeils II;

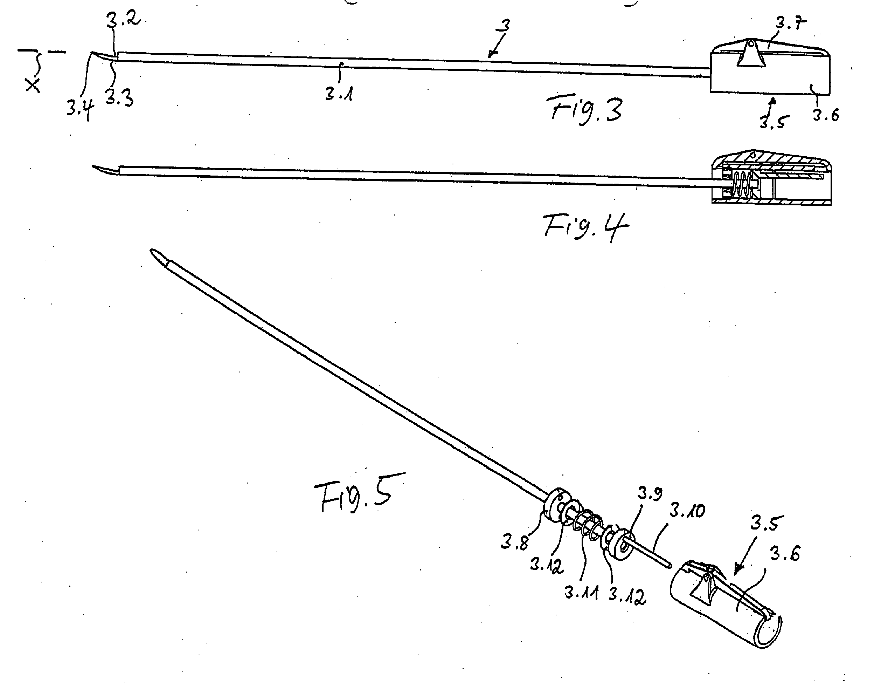

- Fig. 3: eine Seitenansicht eines Führungsteils in Form eines Deflektors der erfindungsgemäßen Werkzeugeinheit;
- Fig. 4: den Deflektor mit im Längsschnitt wiedergegebenen proximalen Verbindungsteil;
- Fig. 5: eine Explosionsdarstellung des Deflektors der Fig. 3, 4;
- Fig. 6: in Nebeneinanderdarstellung eine Führungskanüle und ein Werkzeugteil mit Schaft und Werkzeug der erfindungsgemäßen Werkzeugeinheit;
- Fig. 7: eine vergrößerte Darstellung der Führungskanüle in Seitenansicht, teilweise geschnitten; und

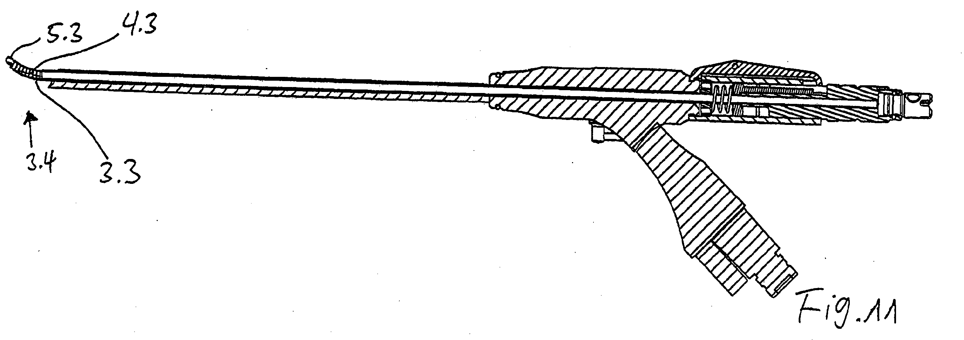

- Fig. 8-Fig. 11: Längsschnittdarstellungen durch eine erfindungsgemäße Werkzeugeinheit, die zusammen mit der Darstellung der Fig. 1 die Abfolge der Bereitstellung des erfindungsgemäßen Werkzeugs zum Einsatz darstellen.

Die erfindungsgemäße chirurgische Werkzeugeinrichtung 1 weist in der dargestellten Ausführungsform ein Endoskop 2, ein Führungsteil 3 in Form eines Deflektors 3, eine Führungskanüle 4 und ein Werkzeugteil 5 auf.

Das Endoskop 2 weist einen gestreckten Arbeitskanal 2.1 mit einer mit diesem fluchtenden proximalen Öffnung 2.2 zum Einführen von Werkzeugen auf. Das Endoskop 2 weist weiterhin, hier nicht näher dargestellt, einen abgewinkelten Beleuchtungseinlass 2.3 und einen parallel zu diesem verlaufenden Beobachtungsauslass 2.4 auf, die mit entsprechenden lichttransparenten Kanälen in Verbindung stehen, wobei an ersterem eine Beleuchtungseinheit und an letzterem eine Beobachtungseinheit, beispielsweise eine Kamera, und über diese ein Bildschirm angeschlossen werden können. Schließlich sind Spüleineinlässe und Spülauslässe 2.5, 2.6 vorgesehen, von denen zumindest einer unmittelbar mit dem Arbeitskanal in Verbindung stehen kann, gegebenenfalls auch beide, bei intermittierendem Spülen und Absaugen. Üblicherweise weist ein Endoskop, wie schon angedeutet, neben dem Arbeitskanal weitere Kanäle, beispielsweise für die optische Übertragung und/oder zum Spülen, auf, die in den Figuren der Übersichtlichkeit halber nicht im Einzelnen dargestellt sind, da es auf diese für den Erfindungsgegenstand nicht im Einzelnen ankommt.

Im Arbeitskanal 2.1 des Endoskops 2 ist ein Deflektorrohr 3.1 des Führungsteils 3 angeordnet. Das Deflektorrohr 3.1 weist eine distale Öffnung 3.2 auf. Es weist über diese hinauserstreckend eine gegenüber der Achse X des Deflektorrohrs 3.1 bzw. des Führungsteils 3 leicht gebogenen einseitigen Führungsabschnitt 3.3 mit im Wesentlichen axialer Erstreckung auf, der von einer Wandungsseite des Deflektorrohrs 3.1 seitlich bogenförmig bis zu einer Radialposition reicht, die in etwa mit der Ausgangsseite des Führungsabschnitts 3.3 diagonal gegenüberliegenden Seite des Deflektorrohrs 3.1 fluchtet. Der Abschnitt 3.3 ist dabei löffelförmig ausgebildet.

Ein Verbindungsteil 3.5 des Führungsrohrs 3 weist einen zylindrischen Mantel 3.6 auf, der als Klinkenhalter für eine wippenartige Klinke 3.7 fungiert, mittels derer der Deflektor 3 sicher am Endoskop 2 festlegbar ist. Am distalen Ende des zylindrischen Mantels 3.6 ist im Inneren desselben eine Buchse 3.8 festgelegt, beispielsweise durch Verkleben, Verschweißen oder Einschrauben; sie kann auch einstückig mit dem Mantel 3.6 hergestellt sein.

Das Deflektorrohr 3.1 ist gleitend durch die Buchse 3.8 hindurchgeführt und seinerseits mit einer Ringscheibe 3.9 fest verbunden. Die Ringscheibe 3.9 weist weiterhin auf ihrer dem Deflektorrohr 3.1 abgewandten Seite einen sich radial versetzt, aber parallel zur Achse X erstreckenden Stift 3.10 auf, dessen Funktion weiter unten erläutert wird.

Zwischen Buchse 3.8 und Ringscheibe 3.9 ist eine Schraubenfeder 3.11 angeordnet, wobei zwischen der Feder 3.11 und jeweils Buchse 3.8 sowie der Ringscheibe 3.9 jeweils eine (Unterleg-)Scheibe aus Metall, vorzugsweise Edelstahl, angeordnet ist, welches Material auch das der Feder 3.11 ist.

Demgemäß kann das Deflektorrohr 3.1 entgegen der Wirkung der Feder 3.11 in distaler Richtung verschoben werden und wird bei Entlastung durch die Feder in beschränktem Umfange in proximaler Richtung verschoben.

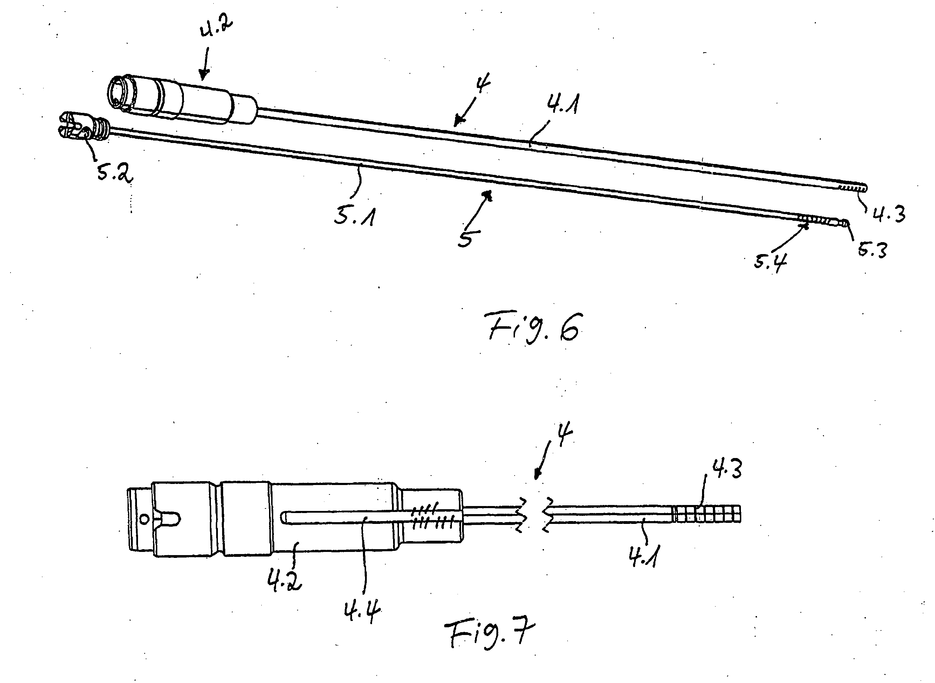

Die Führungskanüle 4 weist einerseits einen metallischen Schaft 4.1 und andererseits an seinem proximalen Ende ein Übergangsteil 4.2 auf (Fig. 6), das in der Fig. 7 deutlicher erkennbar ist.

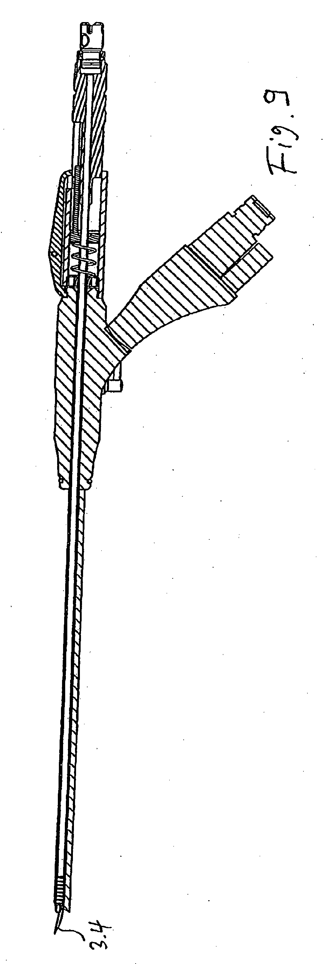

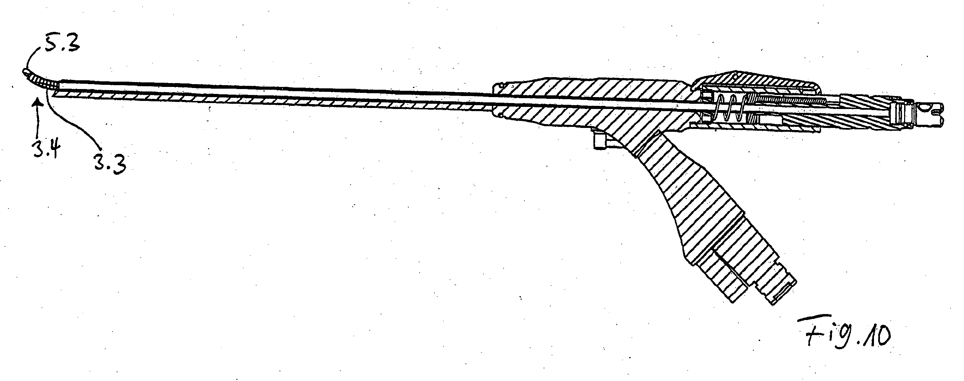

Der distale Endbereich des Schafts 4.1 ist einseitig mit mehreren in parallelen Radialebenen liegenden Schlitzen 4.3 versehen, die sich über mehr als den halben Umfang des Schafts erstrecken, also etwa um 200° bis 270°. Hierdurch wird der Endbereich des Schafts 4.1 zu der Seite der Schlitze hin abbiegbar (Fig. 9, 10).

Da die Abbiegung in die durch den Verlauf des gebogenen löffelförmigen Endbereichs des Deflektors 3 vorgegebene Richtung gewährleistet sein muss, muss sichergestellt sein, dass die Führungskanüle 4 im Deflektorrohr 3.1 in richtiger Ausrichtung einsitzt.

Hierzu dient zum einen der schon erwähnte Stift 3.10 des Deflektors 3 und zum anderen eine am Übergangsteil 4.2 des Führungsrohrs 4 ausgebildete achsparallele Längsnut 4.4, in die beim Einschieben des Schafts 4.1 in das Deflektorrohr 3.1 und des Übergangsteils 4.2 in den Mantel 3.6 des Führungsteils 3 der Stift 3.10 eingreift, so dass das Einschieben der Führungskanüle 4 in den Deflektor 3 nur in der hierdurch bestimmten angularen Ausrichtung erfolgen kann.

Weiter zeigt die Fig. 6 das Werkzeugteil 5 mit einer im Wesentlichen starren Werkzeugwelle 5.1, einem proximalen Kupplungsteil 5.2 und einem Werkzeug 5.3, wie einem Fräserkopf, wobei auch andere rotatorisch einzusetzende Werkzeuge in Frage kommen.

Die Werkzeugwelle 5.1 ist in ihrem distalen Endbereich ebenfalls flexibel ausgebildet und zwar durch eine in der Zeichnung nur angedeutete Wendel 5.4, insbesondere in Form einer Schraubenfeder, bei der die einzelnen Windungen direkt aneinander anliegen.

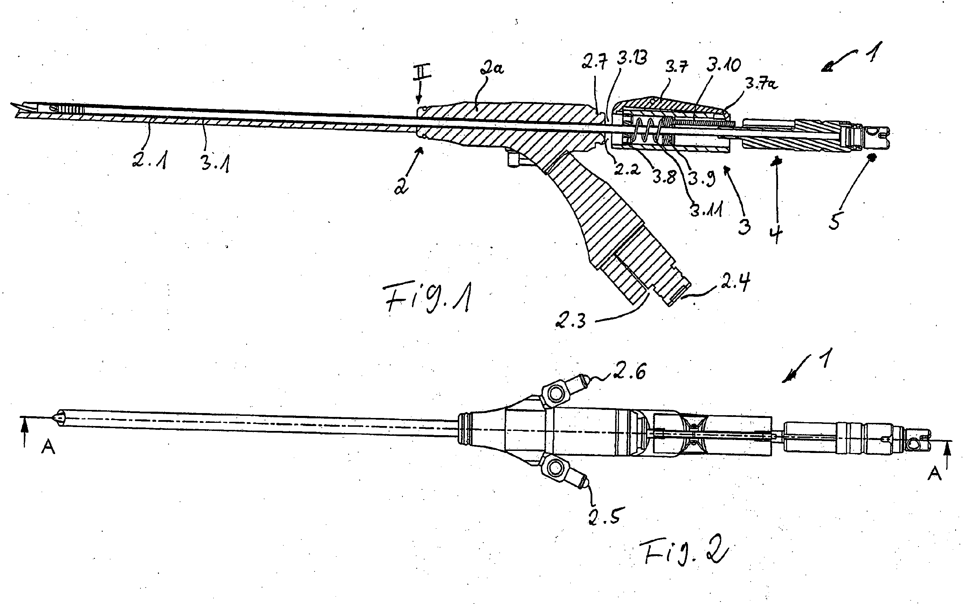

Zum Einsatz der erfindungsgemäßen Werkzeugeinheit wird das Werkzeugteil 5 mit der Werkzeugwelle 5.1 in das Führungsrohr 4 eingeschoben, wie es beispielsweise in der Fig. 1 dargestellt ist, wobei das eigentliche Werkzeug 5.3 das distale Ende des Schafts 4.1 des Führungsrohrs 4 distal überragt, also aus dem Schaft 4.1 herausragt. Werkzeugteil 5 und Führungsrohr 4 sind axial fest, aber drehbar miteinander verbunden und zwar gegebenenfalls direkt über einen in eine Ringnut am distalen Ende des Kupplungsteils 5.2 hineinragenden Übergangsteil 4.2 festgelegten Radialstift (nicht dargestellt). In dieser Position sind Werkzeugteil 5 und Führungsrohr 4 miteinander axial fest, aber relativ zueinander drehbar im proximalen Bereich der Teile 4.2, 5.2 gekoppelt. Die Kopplung kann auch insbesondere über einen einen Drehantrieb aufweisenden Handgriff erfolgen, der letzterer in nicht dargestellter Weise mittels einer Rastverbindung mit dem Verbindungsteil 4.2 des Führungsrohrs 4 axial - und drehfest - verbunden ist, während die Kupplung 5.2 des Werkzeugteils 5 drehfest und über die Verbindung des Verbindungsteils 4.2 mit dem Handgriff auch axial mit der Werkzeugwelle des Antriebs im Handgriff verbunden ist.

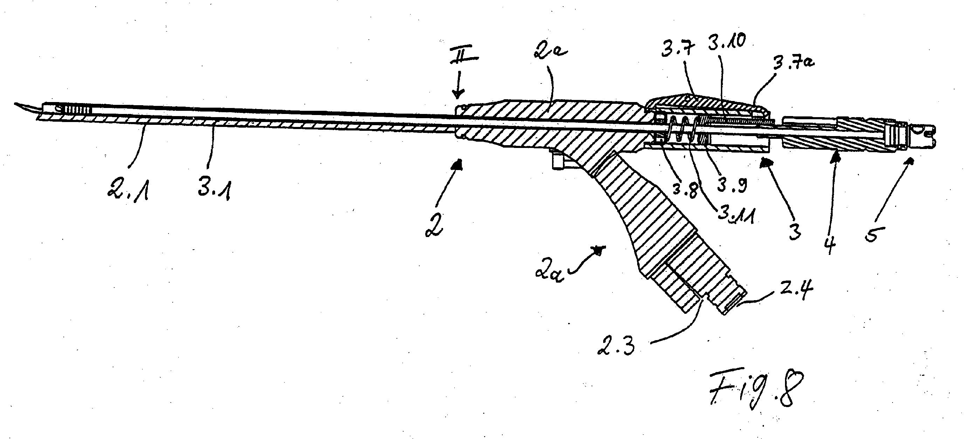

Zur Verbindung von Endoskop 2 und Deflektorrohr 3.1 wird dieses in das Endoskop 2 zunächst so weit eingeschoben, dass die Nase 3.13 der Klinke 3.7 am Deflektor 3 in eine Axialposition gelangt, die mit der der Umfangsnut 2.7 an einem Angriffsteil 2a des Endoskops übereinstimmt. Eine proximale Nase 3.7a der Klinke 3.7 ist breiter als die Nut 4.4, so dass, sobald die Klinke 3.7 mit ihrer proximalen Nase 3.7a über das Übergangsteil 4.2 geschoben wird, die proximale Nase 3.7a der Klinke 3.7 durch die Umfangsfläche des Übergangsteils 4.2 radial angehoben oder nach außen gedrückt wird und so die distale Nase 3.13 der Klinke 3.7 in die Nut 2.7 am Griffteil 2.5 des Endoskops 2 eingedrückt wird (Übergang von Fig. 8 zu Fig. 9), so dass beide damit fest axial gekoppelt sind (Fig. 9).

Die erfindungsgemäße Werkzeugeinheit wird bevorzugt zum Bearbeiten, wie Ausfräsen von Knochenmaterial an Wirbeln der Wirbelsäule eingesetzt. Nach dem minimal-invasiven Herstellen eines Einführungskanals und Einführen des Endoskops bis zum Arbeitsbereich an dem entsprechenden Knochen, insbesondere Wirbel, wird weiterhin zunächst der Deflektor 3 mit dem Deflektorrohr 3.1 mit vollständig eingeschobenem Führungsrohr 4, allerdings ohne Belastung der Feder 3.11 eingeschoben, beispielsweise ausgehend von den in der Fig. 1 dargestellten Relativpositionen zu den aus der Fig. 8 ersichtlichen Relativpositionen der Teile.

Anschließend wird das Führungsrohr 4 so weit vorgeschoben, bis das Übergangsteil 4.2 an der Ringscheibe 3.9 - ebenfalls noch ohne unter Spannung setzen der Feder 3.11 - zur Anlage kommt, wie dies in Fig. 9 dargestellt ist. Dabei verbleibt das Deflektorrohr 3.1 bis zu seiner distalen Öffnung 2.2 innerhalb des Endoskops 2.

Demgegenüber überragen sowohl das Führungsrohr 4 mit dem mit den Schlitzen 4.3 versehenen flexiblen Endbereich als auch das Werkzeugteil 5 mit seinem durch die Wendel 5.4 flexiblen Endbereich. Beide flexiblen Endbereiche werden in dem löffelartigen Führungsabschnitt 3.3 des Deflektors 3 zum Liegen kommen und durch diesen zur Seite gebogen und mit ihm auch das eigentliche Werkzeug 5.3, das im Übrigen die Spitze 3.4 des löffelartigen Führungsabschnitts 3.3 des Deflektors 3 distal überragt. Hierdurch kann das Werkzeug 5.3 nach Einschalten des Drehantriebs frei arbeiten und beispielsweise eine Hohlkehle im Knochenmaterial eines Wirbels erzeugen.

Ein axialer Vorschub und damit ein axiales Vorarbeiten des Werkzeugs wird dadurch bewirkt, dass über den Handgriff des Antriebs Werkzeugteil 5 mit Führungsrohr 4 entgegen der Federwirkung unter Komprimierung der Feder 3.11 in distaler Richtung relativ zum Endoskop 2 bewegt werden (Übergang von Fig. 9 zu Fig. 10), wobei auch das Deflektorrohr 3.1 über die Ringscheibe 3.9 mitgenommen wird.

Nach Beendigung des Arbeitens wird das als Deflektor ausgebildete Führungsteil 3 wieder beim Übergang der Fig. 9 zur Fig. 8 vom Endoskop 2 gelöst, indem beim Herausziehen der Führungskanüle deren Übergangsteil 4.2 die proximale Nase 3.7a der Klinke 3.7 wieder freigibt, so dass sich die distale Nase 3.13 aus der Umfangsnut 2.7 des Endoskops 2 wieder lösen und damit Endoskop 2 und Führungsteil 3 (Deflektor) getrennt werden können.

### Bezugszeichenliste

- 1: Chirurgische Werkzeugeinrichtung
- 2: Endoskop
- 2a: Angriffsteil von 2
- 2.1: Arbeitskanal
- 2.2: Öffnung
- 2.3: Beleuchtungseinlass
- 2.4: Beobachtungsauslass
- 2.5: Spüleinlass
- 2.6: Spülauslass
- 2.7: Umfangsnut
- 3: Führungsteil, insbesondere Deflektor
- 3.1: Rohr von 3
- 3.2: distale Öffnung von 3.1
- 3.3: Führungsabschnitt
- 3.4: Spitze
- 3.5: Verbindungsteil
- 3.6: Mantel
- 3.7: Klinke
- 3.7a: proximale Nase von 3.7
- 3.8: Buchse
- 3.9: Ringscheibe
- 3.10: Stift
- 3.11: Feder
- 3.13: distale Nase von 3.7
- 4: Führungskanüle
- 4.1: Schaft
- 4.2: Übergangsteil
- 4.3: Schlitze
- 4.4: Nut
- 5: Werkzeugteil
- 5.1: Werkzeugwelle
- 5.2: Kupplungsteil
- 5.3: Werkzeug
- 5.4: Wendel

- X: Achse

## Patentansprüche

1. Chirurgische Werkzeugeinrichtung (1), mit einem Endoskop (2), und einem Führungsteil (3) mit einem in diesem geführten Werkzeugteil (5), wobei Führungsteil (3) und Werkzeugteil (5) durch den Arbeitskanal (2.1) des Endoskops (2) mit ihren distalen Enden zu einem distalen Arbeitsbereich verschiebbar sind und wobei das Führungsteil (3) mit dem Endoskop (2) axial fest verbindbar ist, **dadurch gekennzeichnet, dass** das Werkzeugteil (5) eine Werkzeugwelle (5.1) mit einem rotatorischen Werkzeug (5.3) aufweist, dass eine Umfangsnut (2.7) am proximalen Ende des Endoskops (2) ausgebildet ist und am Führungsteil (3) eine zum Eingriff in die Umfangsnut (2.7) beweglich gelagerte Nase (3.13) an dem distalen Ende einer wippenartigen Klinke (3.7) ausgebildet ist und dass bei axial übereinstimmender Anordnung von Umfangsnut (2.7) des Endoskops (2) und der Nase (3.13) des Führungsteils (3) die Nase (3.13) unter Einwirkung eines Übergangsteils (4.2) einer Führungskanüle (4) der Werkzeugeinrichtung in die Umfangsnut (2.7) zur axialen Festlegung von Endoskop (2) und Führungsteil (3) eindrückbar ist.

2. Einrichtung nach Anspruch 1, **gekennzeichnet durch** eine das Werkzeugteil (5) aufnehmende Führungskanüle (4), welche mit dem Werkzeugteil innerhalb des Führungsteils aufnehmbar ist.

3. Einrichtung Anspruch 2, **dadurch gekennzeichnet, dass** Führungskanüle (4) und Werkzeugteil (5) axial fest, aber relativ zueinander drehbar miteinander verbunden sind.

4. Einrichtung nach einem der Ansprüche 2 oder 3 **dadurch gekennzeichnet, dass** Führungsteil (3) und Führungskanüle (4) drehfest zueinander verbindbar sind.

5. Einrichtung nach einem der Ansprüche 2 bis 3, **dadurch gekennzeichnet, dass** die Führungskanüle (4) proximal eine achsparallele Außennut (4.4) und dass das Führungsteil (3) innerhalb eines proximalen Verbindungsteils einen achsparallel gerichteten Stift (3.10) zum Zusammenwirken mit der Nut (4.4) aufweist.

6. Einrichtung nach einem der vorangehenden Ansprüche, **dadurch gekennzeichnet, dass** ein Rohr (3.1) des Führungsteils (3) relativ zu einem es mit dem Endoskop (2) koppelnden Teil (3.6, 3.7) entgegen der Wirkung einer Feder (3.11) axial beweglich ist.

7. Einrichtung nach einem der vorangehenden Ansprüche, **dadurch gekennzeichnet, dass** das Werkzeugteil (5) mit der Abtriebswelle eines Drehantriebs kuppelbar ist.

8. Einrichtung nach einem der Ansprüche 1 bis 7, **dadurch gekennzeichnet, dass** das Führungsteil als Deflektor mit einem an seinem distalen Ende gegenüber seiner Achse (X) seitlich abgebogenen Führungsabschnitt ausgebildet ist.

9. Einrichtung nach Anspruch 8, **dadurch gekennzeichnet, dass** sowohl Führungskanüle (4) als auch Werkzeugteil (5) in ihrem distalen Endbereich flexibel ausgebildet sind.

## Claims

1. A surgical tool device (1), comprising a guiding part (3), a tool part (5); and an endoscope (2) with a working channel (2.1) through which the guiding part (3) and the tool part (5) guiding in same can be moved with their distal ends to a distal working area, wherein the guiding part (3) is rigidly connectable to the endoscope (2) without a relative axial movement, **characterized in that** the tool part (5) has a tool shaft (5.1) with a rotary tool (5.3), that the endoscope has a circumferential groove (2.7) at the proximal end of the endoscope (2) and the guiding part has a movably mounted projection (3.13) formed on the guiding part (3) at the distal end of a rocker catch (3.7) for meshing with the circumferential groove (2.7) and that in case of an axially agreeing arrangement of the circumferential groove (2.7) of the endoscope (2) and of the projection (3.13) of the guiding part (3), the projection (3.13) is pressable into the circumferential groove (2.7) under the action of a transition part (4.2) of a guiding cannula (4) of the device (1) for fixing the endoscope (2) and the guiding part (3) axially and that the guiding part (3) is rigidly connectable with the endoscope (2) axially, whereby when connected, there is no relative movement in an axial direction.

2. A device in accordance with claim 1, **characterized by** a guiding cannula (4), which receives the tool part (5) and which is receivable, with the tool part (5), within the guiding part (3).

3. A device in accordance with claim 2, **characterized in that** the guiding cannula (4) and the tool part (5) are connected to one another axially rigidly but rotatably relative to one another.

4. A device in accordance with claims 2 or 3 , **characterized in that** the guiding part (3) and the guiding cannula (4) are connectable with one another in such a way that they rotate in unison.

5. A device in accordance with claims 2 or 3, **characterized in that** the guiding cannula (4) has an axially parallel outer groove (4.4) proximally and the guiding part (3) has a pin (3.10) directly axially parallel within a proximal connecting part for cooperating with the groove (4.4).

6. A device in accordance with one of the above claims, **characterized in that** the guiding part (3) has a tube (3.1) that is axially movable relative to a coupling part (3.6, 3.7) to the endoscope (2) against the action of a spring (3.11).

7. A device in accordance with one of the above mentioned claims, **characterized in that** the tool part (5) is coupleable with the driven shaft of a rotating drive.

8. A device in accordance with one of the claims 1 to 7, **characterized in that** the guiding part (3) is designed as a deflector with a guiding section bent to the side in relation to its axis (X) at its distal end.

9. A device in accordance with claim 8, **characterized in that** both the guiding cannula (4) and the tool part (5) have a flexible distal end area.

## Revendications

1. Dispositif d'outil chirurgical (1), avec un endoscope (2) et une partie de guidage (3) avec une partie d'outil (5) guidée dans celle-ci, la partie de guidage (3) et la partie d'outil (5) pouvant être coulissées à travers le canal de travail (2.1) de l'endoscope (2) avec leurs extrémités distales orientées vers une zone de travail distale et la partie de guidage (3) pouvant être reliée fixement dans le plan axial à l'endoscope (2), **caractérisé en ce que** la partie d'outil (5) comporte un arbre d'outil (5.1) équipé d'un outil rotatif (5.3), qu'une rainure périphérique (2.7) est réalisée au niveau de l'extrémité proximale de l'endoscope (2) et qu'un bec (3.13) disposé de façon mobile au niveau de la partie de guidage (3) pour s'engrener dans la rainure périphérique (2.7) est réalisé au niveau de l'extrémité distale d'un cliquet (3.7) de type bascule et qu'en présence d'un agencement, coïncidant dans le plan axial, de rainure périphérique (2.7) de l'endoscope (2) et de bec (3.13) de la partie de guidage (3), le bec (3.13) peut être comprimé sous l'effet d'une partie de transition (4.2) d'une canule de guidage (4) du dispositif d'outil dans la rainure périphérique (2.7) en vue de réaliser la fixation axiale de l'endoscope (2) et de la partie de guidage (3).

2. Dispositif selon la revendication 1, **caractérisé par** la présence d'une canule de guidage (4) logeant la partie d'outil (5) et pouvant être reçue avec la partie d'outil à l'intérieur de la partie de guidage.

3. Dispositif selon la revendication 2, **caractérisé en ce que** la canule de guidage (4) et la partie d'outil (5) sont reliées fixement dans le plan axial mais reliées entre elles de façon à pouvoir pivoter les unes par rapport aux autres.

4. Dispositif selon l'une quelconque des revendications 2 ou 3, **caractérisé en ce que** la partie de guidage (3) et la canule de guidage (4) peuvent être reliées entre elles solidairement en rotation.

5. Dispositif selon l'une quelconque des revendications 2 ou 3, **caractérisé en ce que** la canule de guidage (4) comporte dans le plan proximal une rainure extérieure (4.4) parallèle à l'axe et que la partie de guidage (3) comporte une tige (3.10) orientée parallèlement à l'intérieur d'une partie de jonction proximale pour l'interaction avec la rainure (4.4).

6. Dispositif selon l'une quelconque des revendications précédentes, **caractérisé en ce qu'**un tube (3.1) de la partie de guidage (3) est mobile dans le plan axial par rapport à une partie (3.6, 3.7) de couplage avec l'endoscope (2), à l'encontre de l'effet d'un ressort (3.11).

7. Dispositif selon l'une quelconque des revendications précédentes, **caractérisé en ce que** la partie d'outil (5) peut être couplée à l'arbre d'entraînement en sortie d'un entraînement en entrée rotatif.

8. Dispositif selon l'une quelconque des revendications 1 à 7, **caractérisé en ce que** la partie de guidage prend la forme d'un déflecteur avec une section de guidage incurvée en côté au niveau de son extrémité distale par rapport à son axe (X).

9. Dispositif selon la revendication 8, **caractérisé en ce que** tant la canule de guidage (4) que la partie d'outil (5) sont réalisées de façon flexible dans leur zone d'extrémité distale.
